# EUROPÄISCHE PATENTSCHRIFT

(11) **EP 1 383 503 B1**
(45) Veröffentlichungstag und Bekanntmachung des Hinweises auf die Patenterteilung: **15.10.2008**
(21) Anmeldenummer: 02758191.7
(22) Anmeldetag: 18.04.2002
(51) Int. Cl.: A61K 31/505, A61P 25/28, A61P 25/30, A61P 25/32, A61P 25/34

(54) **VERWENDUNG VON DESOXYPEGANIN ZUR BEHANDLUNG VON KRANKHEITSERSCHEINUNGEN DES ZENTRALEN NERVENSYSTEMS AUFGRUND VON INTOXIKATIONEN MIT PSYCHOTROPEN SUBSTANZEN**
USE OF DESOXYPEGANINE FOR TREATING CENTRAL NERVOUS SYSTEM SYMPTOMS RESULTING FROM INTOXICATIONS BY PSYCHOTROPS
UTILISATION DE DESOXYPEGANINE POUR TRAITER DES SYMPTOMES PATHOLOGIQUES DU SYSTEME NERVEUX CENTRAL RESULTANT D'INTOXICATIONS AVEC DES SUBSTANCES PSYCHOTROPES

(30) Priorität: 24.04.2001 DE 10119863
(43) Veröffentlichungstag der Anmeldung: 28.01.2004
(73) Patentinhaber: HF ARZNEIMITTELFORSCHUNG GmbH, 59368 Werne (DE)
(72) Erfinder: OPITZ, Klaus, 48157 Münster (DE); MOORMANN, Joachim, 59368 Werne (DE); HILLE, Thomas, 56567 Neuwied (DE); BECHER, Frank, 56072 Koblenz (DE)
(74) Vertreter: Flaccus, Rolf-Dieter
(86) Internationale Anmeldenummer: PCT/EP2002/004278
(87) Internationale Veröffentlichungsnummer: WO 2002/087553

(56) Entgegenhaltungen:
- DE-A- 19 906 974
- DE-A- 19 906 975
- DE-A- 19 906 978
- DE-A- 19 906 979
- DE-A- 19 924 951
- DE-C- 19 906 977
- VOVIN R YA ET AL: "CORRECTION OF AKINETIC-ABULIC SYNDROME IN SCHIZOPHRENICS USING CHOLINOTROPIC DRUGS" ZHURNAL NEVROPATOLOGII I PSIKHIATRII IMENI S S KORSAKOVA, Bd. 91, Nr. 2, 1991, Seiten 111-115, XP001119113 ISSN: 0044-4588
- TARASYUK V K ET AL: "USE OF DEOXYPEGANINE HYDROCHLORIDE IN THE MULTIMODALITY TREATMENT OF PATIENTS WITH PERIPHERAL NERVOUS SYSTEM DISEASES" VRACHEBNOE DELO, Nr. 3, 1989, Seiten 92-93, XP001119151 ISSN: 0049-6804

## Beschreibung

Die Erfindung betrifft die Verwendung von Desoxypeganin, als freie Base oder als Säureadditionssalz, zur Herstellung eines Arzneimittels zur Behandlung von:
chronischen zerebralen oder zentralnervösen Ausfallserscheinungen oder chronischen psychiatrischen Symptomen, die als Folgeerscheinungen infolge einer Rauschmittel- oder Suchtmittelabhängigkeit oder nach chronischem Rauschmittel- oder Suchtmittelmißbrauch, insbesondere als Folgeerscheinung des chronischen Alkoholmißbrauchs, auftreten und die selbst nach einer abgeschlossenen Entzugstherapie weiter andauern; oder von
chronischen zerebralen oder zentralnervösen Ausfallserscheinungen oder chronischen psychiatrischen Symptomen, die als Folgeerscheinungen infolge von Medikamentenmißbrauch beim Menschen auftreten; oder von
zerebralen, zentralnervösen oder psychiatrischen Symptomen, Ausfallserscheinungen oder Krankheiten, die als Nebenwirkungen bei bestimmungsgemäßer, insbesondere bei wiederholter oder länger andauernder Anwendung von Arzneimitteln, beim Menschen oder anderen Wirbeltieren auftreten; oder von
zerebralen, zentralnervösen oder psychiatrischen Symptomen, Ausfallserscheinungen oder Krankheiten, die infolge einer akuten Vergiftung durch psychotrope toxische Substanzen beim Menschen oder anderen Wirbeltieren auftreten;
zerebrale, zentralnervöse oder psychiatrische Symptome, Ausfallserscheinungen oder Krankheiten, die infolge chronischer Einwirkung von psychotrop wirkenden toxischen Substanzen beim Menschen oder anderen Wirbeltieren auftreten;
wobei es sich bei den genannten Symptomen, Ausfallserscheinungen oder Krankheiten um kognitive Störungen handelt.

Die Zuführung von psychotropen Substanzen, auch von Rauschmitteln, insbesondere von Alkohol, führt bekanntermaßen zu Symptomen wie Wahrnehmungsstörungen, Gedächtnisverlust, Beeinträchtigung kognitiver Fähigkeiten, allgemeinem Kontrollverlust, Aggressivität, Beeinträchtigung der Bewegungskoordination etc..
Wird die Substanz als Rauschmittel, wie z. B. Heroin, Kokain oder als ethylalkoholhaltiges Getränk, bewußt zugeführt, so sind derartige Wirkungen zwar von der rauschmittelkonsumierenden Person beabsichtigt, jedoch werden sie unter bestimmten Voraussetzungen auch als nachteilig empfunden. Hinzu kommt, daß der Schweregrad und die zeitliche Fortdauer dieser Symptome variieren kann und für den Rauschmittelkonsumenten im voraus oft nur schwer einzuschätzen ist.

Insbesondere bei chronischer Abhängigkeit und fortgesetztem Suchtmittelmißbrauch kommt es nicht nur zu den allgemein bekannten Organschädigungen, sondern es treten auch anhaltende Ausfallserscheinungen auf, welche z. B. die kognitiven Leistungen, insbesondere Gedächtnisleistungen, beeinträchtigen. ständen führen. Auch die bereits genannten psychiatrischen Symptome, wie z. B. ein allgemeiner Kontrollverlust, können sich chronisch manifestieren. Diese chronischen Folgeerscheinungen des Alkoholmißbrauchs - die bei anderen Suchtmittelabhängigkeiten in ähnlicher Weise auftreten - stellen ein erhebliches Hindernis für eine erfolgreiche Durchführung von Entwöhnungstherapien dar. So ist bekannt, daß der durch chronischen Alkoholmißbrauch bedingte Kontrollverlust eine Abstinenz-Unfähigkeit des betroffenen Alkoholkranken bewirkt. Dies ist die Hauptursache dafür, daß selbst entwöhnte Alkoholiker zu Rückfällen neigen, mit meist schwerwiegenden Folgen. Aus dieser Beobachtung wurde der Grundsatz abgeleitet, daß ein "kontrolliertes Trinken" bei Abhängigen nicht möglich ist.

Es ist ferner bekannt, daß es hinsichtlich des Rauschmittelkonsumverhaltens große individuelle Unterschiede gibt, aufgrund derer man beispielsweise Alkoholiker in unterschiedliche Trinker-Kategorien einteilt.
Bei bestimmten Alkohol-Konsumenten besteht das Problem, daß nach Überschreiten einer bestimmten individuellen Schwellendosis rasch ein allgemeiner Kontrollverlust mit den oben erwähnten negativen Begleiterscheinungen eintritt. Die betroffenen Personen sind meist nicht in der Lage, das Erreichen ihrer individuellen Schwellendosis oder überhaupt ihr persönliches Rückfall-Risiko rechtzeitig zu erkennen. Aufgrund des dadurch bewirkten Kontrollverlustes, der häufig zu weiterem, exzessiven Alkoholgenuß führt, werden solche Personen oft als "dangerous drinkers" bezeichnet. Häufig handelt es sich dabei um Personen, die bereits Entzugstherapien hinter sich haben und auf diese Weise rückfällig werden.

Bekanntermaßen hat der durch chronischen Suchtmittelmißbrauch verursachte Kontrollverlust, wie auch die Beeinträchtigung der Gedächtnisleistungen (bis hin zur Demenz) für den Betroffenen wie auch für seine Umgebung oft weiterreichende Folgen, wie z. B. Unfähigkeit zur Berufsausübung, Unfähigkeit zur Strukturierung des Tagesablaufs, Unfähigkeit zur Aufnahme und Pflege sozialer Kontakte und daraus folgend soziale Ausgrenzung.
Diese Ausfallserscheinungen, z. B. die Beeinträchtigung der kognitiven Leistungen, dauern selbst nach einer erfolgreich abgeschlossenen Entzugstherapie oft noch an. Weitere bei Alkoholmißbrauch oder bei Mißbrauch anderer Suchtmittel auftretende psychiatrische oder zerebrale Störungen sind z. B.: Wahrnehmungs- oder Sinnestäuschungen, Amnesie, Bewußtseinsveränderungen, formale Denkstörungen, Merkfähigkeitsstörungen, Wahnvorstellungen, Konfabulationen, Desorientiertheit, Erregungszustände.

Es bestand daher die Aufgabe, die infolge chronischen Suchtmittelmißbrauchs, insbesondere Alkoholmißbrauchs, auftretenden psychiatrischen oder zentralnervös verursachten Symptome, insbesondere den Kontrollverlust, Verlust kognitiver Fähigkeiten, Demenz etc. zu beseitigen oder zumindest abzumildern.

Zur Lösung dieses Problems wird nach der Erfindung vorgeschlagen, zur Behandlung von Personen, die an derartigen Folgeerscheinungen von Suchtmittelabhängigkeiten leiden, den Wirkstoff Desoxypeganin zu verwenden. Durch Verabreichung von Desoxypeganin kann bewirkt werden, daß die infolge von chronischem Alkohol- oder Rauschmittelgenuß verursachten psychiatrischen oder zerebralen Krankheitserscheinungen, insbesondere der eingetretene Verlust kognitiver Fähigkeiten bzw. Kontrollverlust, zumindest teilweise aufgehoben bzw. rückgängig gemacht werden, so daß die genannten Fähigkeiten nach und nach wiedererlangt werden. Somit können gemäß vorliegender Erfindung bestimmte chronische Symptome von Suchtmittelabhängigkeiten beseitigt oder zumindest abgemildert werden.

Die Erfindung beruht auf der überraschenden Beobachtung, daß im Tierversuch bei Ratten durch Verabreichung von Desoxypeganin eine Wiedererlangung der kognitiven Fähigkeiten herbeigeführt werden konnte. Bei nicht behandelten Kontrolltieren trat diese Wiederherstellung nicht oder erst wesentlich später ein.

Desoxypeganin (1,2,3,9-Tetrahydropyrrolo[2,1-b]chinazolin) ist ein Alkaloid der Summenformel C₁₁H₁₂N₂, das in Pflanzen aus der Familie der Zygophyllaceae enthalten ist. Die Gewinnung von Desoxypeganin erfolgt vorzugsweise durch Isolierung aus der Steppenraute (Peganum harmala) oder durch Synthese.

Aufgrund seiner pharmakologischen Eigenschaften wird Desoxypeganin zur Gruppe der reversibel wirkenden Cholinesterasehemmstoffe gezählt. Daneben wirkt es auch als Monoaminoxidase-Hemmer.
Bezüglich der Verwendung in der medikamentösen Therapie wurde vorgeschlagen, Desoxypeganin zur Behandlung der Alzheimer' schen Demenz, zur Behandlung des Alkoholismus durch Unterdrückung des Verlangens nach Alkohol, zur Behandlung der Nikotinabhängigkeit durch Verminderung des Verlangens nach Nikotin oder zur Substitutionstherapie von Drogensüchtigen bzw. zur Behandlung von Entzugserscheinungen während einer Entzugstherapie. Außerdem kann Desoxypeganin als Cholinesterasehemmer als Antidot oder Prophylaktikum bei Vergiftungen durch organische Phosphorsäureester eingesetzt werden, wobei es die zerebrale Wirkung cholinerger Gifte antagonisiert.

Nach der Erfindung kann Desoxypeganin sowohl in Form seiner freien Base als auch als Säureadditionssalz zur Behandlung verwendet werden; als Salze werden Desoxypeganinhydrochlorid und Desoxypeganinhydrobromid bevorzugt. Daneben können auch Salze anderer pharmakologisch akzeptabler Säuren verwendet werden, z. B. Citrat, Tartrat oder Acetat.

Desoxypeganin wird vorzugsweise in einer Arzneizubereitung verabreicht, welche den Wirkstoff in Anteilen von 0,1 bis 90 Gew.-%, besonders bevorzugt in Anteilen von 2 bis 20 Gew.-%, enthält, jeweils berechnet als freies Desoxypeganin. Die erfindungsgemäß verwendeten Desoxypeganinhaltigen Arzneizubereitungen können darüber hinaus Hilfsstoffe, Trägerstoffe, Stabilisatoren etc. in den dem Fachmann bekannten Mengen enthalten.
Die täglich verabreichte Dosis liegt vorzugsweise im Bereich von 0,1 bis 100 mg, insbesondere von 10 bis 50 mg. Sie ist in Abhängigkeit von den individuellen Voraussetzungen entsprechend einzustellen.

Die Zubereitungen, welche gemäß vorliegender Erfindung zur Verabreichung von Desoxypeganin verwendet werden, können einen oder mehrere folgender Zusatzstoffe enthalten:
- Antioxydantien, Synergisten, Stabilisatoren;
- Konservierungsmittel;
- Geschmackskorrigentien;
- Färbemittel;
- Lösemittel, Lösungsvermittler;
- Tenside (Emulgatoren, Solubilisatoren, Benetzer, Entschäumer);
- Viskositäts- und Konsistenzbeeinflusser, Gelbildner;
- Resorptionsbeschleuniger;
- Adsorptionsmittel, Feuchthaltemittel, Gleitmittel;
- Zerfalls- und Lösungsbeeinflusser, Füllmittel (Streckmittel), Peptisatoren;
- Freisetzungsverzögerer.

Diese Aufzählung ist nicht abschließend; die in Frage kommenden physiologisch unbedenklichen Substanzen sind dem Fachmann bekannt.

Die Verabreichung von Desoxypeganin kann oral oder parenteral erfolgen. Für die orale Verabreichung können bekannte Darreichungsformen wie Tabletten, Dragees oder Pastillen verwendet werden. Daneben kommen auch flüssige oder halbflüssige Darreichungsformen in Betracht, wobei der Wirkstoff als Lösung oder Suspension vorliegt. Als Löse- oder Suspendierungsmittel können Wasser, wässrige Medien oder pharmakologisch unbedenkliche Öle (vegetabilische oder Mineralöle) verwendet werden. Vorzugsweise sind die Desoxypeganin enthaltenden Arzneimittel als Depot-Arzneimittel formuliert, welche in der Lage sind, diesen Wirkstoff über einen längeren Zeitraum in kontrollierter Weise an den Organismus abzugeben.

Darüber hinaus kann die Verabreichung von Desoxypeganin nach der Erfindung auch auf parenteralem Wege erfolgen. Hierzu können transdermale oder transmucosale Darreichungsformen besonders vorteilhaft für die erfindungsgemäße Verabreichung von Desoxypeganin verwendet werden, insbesondere klebende transdermale therapeutische Systeme (Wirkstoffpflaster). Diese ermöglichen es, den Wirkstoff über einen längeren Zeitraum in kontrollierter Weise über die Haut an den zu behandelnden Patienten abzugeben.
Ein weiterer Vorteil ist, daß eine mißbräuchliche Verwendung bei parenteralen Applikationsformen weniger leicht möglich ist als bei oralen Darreichungsformen. Durch die vorgegebene Wirkstoff-Freisetzungsfläche und die vorbestimmte Freisetzungsrate kann eine Überdosierung seitens des Patienten weitgehend ausgeschlossen werden. Außerdem sind transdermale Darreichungsformen aufgrund weiterer Eigenschaften sehr vorteilhaft, z. B. Vermeidung des First-Pass-Effektes oder eine bessere, gleichmässigere Steuerung des Blutspiegels.

Derartige transdermale, Desoxypeganin enthaltenden Systeme weisen üblicherweise eine wirkstoffhaltige, haftklebende Polymermatrix auf, die auf der hautfernen Seite von einer wirkstoffundurchlässigen Rückschicht bedeckt ist, und deren klebende, wirkstoffabgebende Oberfläche vor der Applikation mit einer ablösbaren Schutzschicht bedeckt ist. Die Herstellung solcher Systeme und die dabei verwendbaren Grundstoffe und Hilfsstoffe sind dem Fachmann grundsätzlich bekannt; beispielsweise wird der Aufbau solcher transdermaler therapeutischer Systeme in den deutschen Patenten DE 33 15 272 und DE 38 43 239 oder in den US-Patenten 4 769 028, 5 089 267, 3 742 951, 3 797 494, 3 996 934 und 4 031 894 beschrieben.
Ein erfindungsgemäß verwendetes transdermales therapeutisches System (TTS) kann einen Gehalt von 0,1 bis 50 Gew.-% Desoxypeganin, besonders bevorzugt von 2 bis 20 Gew.-%, in der Matrix bzw. im Wirkstoffreservoir aufweisen. Geeignete Desoxypeganin enthaltende TTS sind beispielsweise in WO 00/48579 beschrieben.

Als parenterale Darreichungsformen kommen ferner auch Injektionslösungen in Betracht, insbesondere solche, die eine Depotwirkung bzw. verzögerte und anhaltende Freisetzung des Wirkstoffs ermöglichen. Die hierfür geeigneten Formulierungen sind dem Fachmann bekannt, z. B. Formulierungen auf nichtwässriger Basis (z. B. auf der Basis von physiologisch verträglichen Öle).

Durch die vorliegende Erfindung wird es möglich, bestimmte Begleit- oder Folgeerscheinungen eines chronischen Suchtmittelmißbrauchs zu behandeln, wodurch das Allgemeinbefinden dieser Patienten verbessert wird und die soziale Wiedereingliederung von chronisch Suchtmittelgeschädigten unterstützt wird. Außerdem werden durch die erfindungsgemäß vorgeschlagene Behandlung mit Desoxypeganin die Erfolgsaussichten von Entzugstherapien verbessert und das Rückfallrisiko vermindert. Die Behandlung fördert zudem die soziale Reintegration der betroffenen Personen.

Die beschriebenen psychiatrischen oder zerebralen Störungen, insbesondere eine Beeinträchtigung der kognitiven Leistungen oder Demenz, können auch infolge der Zuführung oder des Mißbrauchs anderer Wirkstoffe, wie Umweltgifte (PCB, Dioxine, Furane, Pentachlorphenol, Quecksilber- und Bromverbindungen, Amalgam, chlorierte Kohlenwasserstoffe wie bestimmte Lösungsmittel), durch Sucht- oder Rauschmittel, oder infolge von Medikamentengebrauch oder -mißbrauch auftreten.

Unter Wirkstoffen werden im Sinne der Erfindung deshalb grundsätzlich alle psychotropen Substanzen, seien sie fest, flüssig, dampf- oder gasförmig, verstanden, bei deren einmaligem, gelegentlichem, häufigem oder chronischem Mißbrauch Krankheitserscheinungen der genannten Art auftreten. Neben dem bereits genannten Ethylalkohol kommen auch Methanol und andere Alkohole in Betracht, die beispielsweise als Verunreinigungen in alkoholischen Getränken vorhanden sein können. Insbesondere bezieht sich die Erfindung auf folgende psychotropen Wirkstoffe und wirkstoffhaltigen Zubereitungen, z. B. Pharmaka wie: Neuroleptika, Antidepressiva, Tranquilizer (insbesondere Benzodiazepine), Antipsychotika, Hypnotika, Psychostimulantien (insbesondere Amphetamine, "Modedrogen" wie z. B. "Ecstacy", "Speed" mit unstandardisierten Wirkstoffgemischen), weitere psychotrope Drogen und Substanzen sowie synthetische Derivate davon (z. B. auf der Basis von Johanniskraut, Baldrian, Hopfen, Melisse, Lavendel, Kava-Kava, Absinth; ferner THC-haltige Rauschmittel wie Marihuana und Haschisch, weiterhin Kokain, "Crack", LSD, Psilocybin, Mescalin, Opium, Morphium und Morphinderivate wie Heroin, Codein, Methadon), Holzschutzmittel wie z. B. Chrom(VI)-haltige Holzschutzmittel, sowie bestimmte Lösungsmittel und halogenierte Kohlenstoff- oder Kohlenwasserstoffverbindungen, welche unbeabsichtigt zugeführt oder als Rauschmittel durch "Schnüffeln" konsumiert werden, sowie Umweltgifte (PCB, Dioxine, Furane, Pentachlorphenol, Quecksilber- und Bromverbindungen, Quecksilber und Amalgame).

Auch bei der bestimmungsgemäßen Anwendung einiger der vorstehend genannten Substanzen, die für Therapiezwecke eingesetzt werden, kann es im Rahmen einer ärztlich verordneten Therapie, insbesondere bei wiederholter oder über längere Zeit erfolgender Verabreichung, zu den genannten Nebenwirkungen kommen, z. B. kognitive Störungen, zerebrale Ausfallserscheinungen, psychiatrische Symptome etc..
Es wird deshalb gemäß vorliegender Erfindung vorgesehen, zur Behandlung solcher Störungen, welche durch die bestimmungsgemäße oder mißbräuchliche Anwendung der vorstehend genannten Substanzen verursacht wurden, den Wirkstoff Desoxypeganin zu verabreichen. Das gleiche gilt für Intoxikationen durch die genannten Umweltgifte.

Es ist ferner bekannt, daß die genannten Nebenwirkungen nicht nur durch Gebrauch oder Mißbrauch von psychotropen Substanzen verursacht werden können, sondern auch infolge einmaliger, mehrmaliger oder chronischer Verabreichung anderer Arzneimittel. Die erfindungsgemäße Verwendung von Desoxypeganin erstreckt sich deshalb auch auf die Behandlung von Symptomen oder Nebenwirkungen, die auf diese Weise verursacht wurden.

Auch bei akuten Vergiftungsfällen (z. B. Chemie-Unfälle) sowie bei chronischer Gifteinwirkung (z. B. Umweltgifte wie Holzschutzmittel, PCB, Dioxine, Furane, Pentachlorphenol, Quecksilber- und Bromverbindungen, Quecksilber, Amalgame, chlorierte Kohlenwasserstoffe, halogenierte Biphenyle, Tributylzinn, Holzschutzmittel etc.) werden zerebrale Störungen oder psychiatrische Symptome, z. B. Gedächtnisverlust oder Denkstörungen, bei den betroffenen Personen beobachtet. Die erfindungsgemäße Verwendung von Desoxypeganin erstreckt sich deshalb auch auf die Behandlung von Personen, welche auf die vorstehend genannte Art durch Gifteinwirkung beeinträchtigt wurden.

Schließlich wird gemäß vorliegender Anmeldung auch vorgeschlagen, Desoxypeganin in den oben genannten Fällen an andere Wirbeltiere, insbesondere an Säugetiere, welche an den vorstehend beschriebenen Symptomen oder Störungen leiden, zu verabreichen.

## Patentansprüche

1. Verwendung von Desoxypeganin, als freie Base oder als Säureadditionssalz, zur Herstellung eines Arzneimittels zur Behandlung von:
chronischen zerebralen oder zentralnervösen Ausfallserscheinungen oder chronischen psychiatrischen Symptomen, die als Folgeerscheinungen infolge einer Rauschmittel- oder Suchtmittelabhängigkeit oder nach chronischem Rauschmittel- oder Suchtmittelmißbrauch, insbesondere als Folgeerscheinung des chronischen Alkoholmißbrauchs, auftreten und die selbst nach einer abgeschlossenen Entzugstherapie weiter andauern; oder von
chronischen zerebralen oder zentralnervösen Ausfallserscheinungen oder chronischen psychiatrischen Symptomen, die als Folgeerscheinungen infolge von Medikamentenmißbrauch beim Menschen auftreten; oder von
zerebralen, zentralnervösen oder psychiatrischen Symptomen, Ausfallserscheinungen oder Krankheiten, die als Nebenwirkungen bei bestimmungsgemäßer, insbesondere bei wiederholter oder länger andauernder Anwendung von Arzneimitteln, beim Menschen oder anderen Wirbeltieren auftreten; oder von
zerebralen, zentralnervösen oder psychiatrischen Symptomen, Ausfallserscheinungen oder Krankheiten, die infolge einer akuten Vergiftung durch psychotrope toxische Substanzen beim Menschen oder anderen Wirbeltieren auftreten;
zerebrale, zentralnervöse oder psychiatrische Symptome, Ausfallserscheinungen oder Krankheiten, die infolge chronischer Einwirkung von psychotrop wirkenden toxischen Substanzen beim Menschen oder anderen Wirbeltieren auftreten;
wobei es sich bei den genannten Symptomen, Ausfallserscheinungen oder Krankheiten um kognitive Störungen handelt.

2. Verwendung nach Anspruch 1, **dadurch gekennzeichnet, daß** die mit dem Arzneimittel zu verabreichende Dosis im Bereich von 0,1 bis 100 mg, vorzugsweise 10 bis 50 mg, pro Tag liegt.

3. Verwendung nach Anspruch 1 oder 2, **dadurch gekennzeichnet, daß** es sich bei dem Suchtmittelmißbrauch um Alkoholmißbrauch handelt.

4. Verwendung nach Anspruch 1 oder 2, **dadurch gekennzeichnet, daß** es sich bei dem Suchtmittelmißbrauch um den Mißbrauch von psychotropen Substanzen, insbesondere von halluzinogenen Substanzen, handelt.

5. Verwendung nach einem der Ansprüche 1 bis 4, **dadurch gekennzeichnet, daß** die infolge von Alkoholmißbrauch oder infolge des Mißbrauchs anderer Suchtmittel auftretenden zerebralen oder psychiatrischen Störungen aus der Gruppe ausgewählt sind, die Beeinträchtigungen der Gedächtnisleistungen, Wahrnehmungs- und Sinnestäuschungen, Amnesie, Bewußtseinsveränderungen, formale Denkstörungen, Merkfähigkeitsstörungen, Wahnvorstellungen, Konfabulationen und Desorientiertheit umfaßt.

6. Verwendung nach einem der Ansprüche 1 bis 5, **dadurch gekennzeichnet, daß** das genannte Arzneimittel den Wirkstoff Desoxypeganin in Anteilen von 0,1 bis 90 Gew.-%, vorzugsweise 2 bis 20 Gew.-%, berechnet als freies Desoxypeganin, enthält.

7. Verwendung nach Anspruch 6, **dadurch gekennzeichnet, daß** das genannte Arzneimittel eine Depotwirkung aufweist.

8. Verwendung nach Anspruch 6 oder 7, **dadurch gekennzeichnet, daß** das genannte Arzneimittel ein oral verabreichbares Arzneimittel ist.

9. Verwendung nach Anspruch 6 oder 7, **dadurch gekennzeichnet, daß** das genannte Arzneimittel ein parenteral verabreichbares Arzneimittel ist.

10. Verwendung nach Anspruch 9, **dadurch gekennzeichnet, daß** das genannte Arzneimittel ein transdermal verabreichbares Arzneimittel ist.

## Claims

1. Use of deoxypeganine, as free base or as acid addition salt, for the production of a medicament for the treatment of:
chronic cerebral or central nervous defunctionalization manifestations or chronic psychiatric symptoms occurring as sequelae as a result of addiction to intoxicants or addiction to addictive substances, or after chronic abuse of intoxicants or addictive substances, particularly as a sequela of chronic alcohol abuse, and which persist even after a completed withdrawal therapy; or of
chronic cerebral or central nervous defunctionalization manifestations or chronic psychiatric symptoms occurring as sequelae as a result of the abuse of medicaments in humans; or of
cerebral, central nervous or psychiatric symptoms, defunctionalization manifestations or diseases occurring as side effects of the use of medicaments as intended, more particularly the repeated or prolonged use of medicaments, in humans or other vertebrates; or of
cerebral, central nervous or psychiatric symptoms, defunctionalization manifestations or diseases occurring as a result of acute poisoning by psychotropic toxic substances in humans or other vertebrates;
cerebral, central nervous or psychiatric symptoms, defunctionalization manifestations or diseases occurring as a result of chronic exposure to psychotropically active toxic substances, in humans or other vertebrates;
wherein the aforementioned symptoms, defunctionalization manifestations or diseases are cognitive disturbances.

2. Use according to claim 1, **characterized in that** the dose administered with the medicament is in the range from 0.1 to 100 mg, preferably 10 to 50 mg, per day.

3. Use according to claim 1 or 2, **characterized in that** said abuse of addictive substances is the abuse of alcohol.

4. Use according to Claim 1 or 2, **characterized in that** said abuse of addictive substances is the abuse of psychotropic substances, especially of hallucinogenic substances.

5. Use according to any one of claims 1 to 4, **characterized in that** the cerebral or psychiatric disorders occurring as a result of alcohol abuse or as a result of the abuse of other addictive substances are selected from the group which comprises impairment of memory performance, perceptual illusions or hallucinations, amnesia, alterations of consciousness, formal cognitive disturbances, memory deficits, delusions, confabulations and disorientation.

6. Use according to any one of claims 1 to 5, **characterized in that** said medicament contains the active agent deoxypeganine in proportions of from 0.1 to 90% by weight, preferably 2 to 20% by weight, calculated as free deoxypeganine.

7. Use according to Claim 6, **characterized in that** said medicament has a depot effect.

8. Use according to Claim 6 or 7, **characterized in that** said medicament is administered orally.

9. Use according to Claim 6 or 7, **characterized in that** said medicament is administered parenterally.

10. Use according to Claim 9, **characterized in that** said medicament is administered transdermally.

## Revendications

1. Utilisation de désoxypéganine, sous la forme d'une base libre ou sous la forme d'un sel d'addition d'acide, pour la préparation d'un médicament destiné au traitement :
de phénomènes de défaillances chroniques du cerveau ou du système nerveux central ou de symptômes psychiatriques chroniques, qui se manifestent sous la forme de séquelles d'une dépendance à des stupéfiants ou à des substances addictives ou bien après un abus chronique de stupéfiants ou de substances addictives, en particulier sous la forme de séquelles de l'abus chronique d'alcool, et qui perdurent même après l'achèvement d'une thérapie de sevrage ; ou bien
de phénomènes de défaillances chroniques du cerveau ou du système nerveux central ou de symptômes psychiatriques chroniques, qui se manifestent sous la forme de séquelles d'un abus de médicaments chez l'être humain ; ou bien
de symptômes, de phénomènes de défaillances ou de maladies du cerveau, du système nerveux central ou psychiatriques, qui se manifestent sous la forme d'effets secondaires dans le cas d'une utilisation de médicaments conforme aux prescriptions, en particulier d'une telle utilisation répétée ou de longue durée, chez l'être humain ou chez d'autres vertébrés ; ou bien
de symptômes, de phénomènes de défaillances ou de maladies du cerveau, du système nerveux central ou psychiatriques, qui se manifestent suite à une intoxication aiguë par des substances toxiques psychotropes chez l'homme ou chez d'autres vertébrés ;
de symptômes, de phénomènes de défaillances ou de maladies du cerveau, du système nerveux central ou psychiatriques, qui se manifestent suite à une action chronique de substances toxiques à effet psychotrope chez l'homme ou chez d'autres vertébrés ;
lesdits symptômes, phénomènes de défaillances ou maladies mentionnés ci-dessus étant des troubles cognitifs.

2. Utilisation selon la revendication 1, **caractérisée en ce que** la dose à administrer avec le médicament se situe dans la plage de 0,1 à 100 mg, de préférence de 10 à 50 mg, par jour.

3. Utilisation selon la revendication 1 ou 2, **caractérisée en ce que,** en ce qui concerne l'abus de substances addictives, il s'agit de l'abus d'alcool.

4. Utilisation selon la revendication 1 ou 2, **caractérisée en ce que**, en ce qui concerne l'abus de substances addictives, il s'agit de l'abus de substances psychotropes, en particulier de substances hallucinogènes.

5. Utilisation selon l'une quelconque des revendications 1 à 4, **caractérisée en ce que** les troubles cérébraux ou psychiatriques qui se manifestent suite à un abus d'alcool ou suite à l'abus d'autres substances addictives sont choisis parmi le groupe qui comprend des altérations de la mémoire, des illusions de la perception et des sens, l'amnésie, des troubles de la conscience, des troubles du cours de la pensée, des troubles de la capacité de rétention, des obsessions, des bavardages et une désorientation.

6. Utilisation selon l'une quelconque des revendications 1 à 5, **caractérisée en ce que** le médicament mentionné contient la substance active désoxypéganine dans des fractions de 0,1 à 90 % en poids, de préférence de 2 à 20 % en poids, calculés sous la forme de désoxypéganine libre.

7. Utilisation selon la revendication 6, **caractérisée en ce que** le médicament mentionné présente un effet retard.

8. Utilisation selon la revendication 6 ou 7, **caractérisée en ce que** le médicament mentionné est un médicament qui peut être administré par voie orale.

9. Utilisation selon la revendication 6 ou 7, **caractérisée en ce que** le médicament mentionné est un médicament qui peut être administré par voie parentérale.

10. Utilisation selon la revendication 9, **caractérisée en ce que** le médicament mentionné est un médicament qui peut être administré par voie transdermique.
